(19) Europäisches Patentamt
European Patent Office
Office européen des brevets

(11) **EP 3 319 453 B1**

(12) **EUROPEAN PATENT SPECIFICATION**

(45) Date of publication and mention
of the grant of the patent:
**01.09.2021 Bulletin 2021/35**

(51) Int Cl.:
**A23C 9/15** *(2006.01)*    **A01J 11/10** *(2006.01)*
**G01N 33/06** *(2006.01)*

(21) Application number: **16734390.4**

(22) Date of filing: **04.07.2016**

(86) International application number:
**PCT/EP2016/065662**

(87) International publication number:
**WO 2017/005676 (12.01.2017 Gazette 2017/02)**

(54) **METHOD AND DEVICE FOR IN-LINE FAT STANDARDIZATION OF A DAIRY PRODUCT**

VERFAHREN UND VORRICHTUNG ZUR INLINE-FETTSTANDARDISIERUNG EINES MOLKEREIPRODUKTS

PROCÉDÉ ET DISPOSITIF DE STANDARDISATION EN LIGNE DE LA TENEUR EN GRAISSES D'UN PRODUIT LAITIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2015 SE 1551000**

(43) Date of publication of application:
**16.05.2018 Bulletin 2018/20**

(73) Proprietor: **Tetra Laval Holdings & Finance S.A.**
**1009 Pully (CH)**

(72) Inventors:
• **AVERGÅRD, Pontus**
**S-211 33 Malmö (SE)**
• **ÖHLUND, Åke**
**S-22467 Lund (SE)**

(74) Representative: **Tetra Pak - Patent Attorneys SE**
**AB Tetra Pak**
**Patent Department**
**Ruben Rausings gata**
**221 86 Lund (SE)**

(56) References cited:
WO-A1-90/00862      WO-A1-99/57988
DE-A1- 3 238 462     US-A- 3 946 113
US-A- 3 983 257      US-A- 5 137 738
US-A- 5 983 709

• **Yokogawa: "Stable and accurate density measurement to control fat content in skim milk", Application note vigilant plant, 1 January 2011 (2011-01-01), pages 1-2, XP055299745, Retrieved from the Internet: URL:https://web-material3.yokogawa.com/AN1 0T01K01-02E.pdf [retrieved on 2016-09-05]**
• **3.6.5: "Entrahmen und Fettgehaltseinstellung" In: Heinz G. Kessler: "Lebensmittel- und Bioverfahrenstechnik - Molkereitechnologie", 1 January 1996 (1996-01-01), Verlag A. Kessler, XP002761441, ISBN: 3980237842 vol. 4, pages 49-50, page 49, right-hand column - page 50, left-hand column figures 3.10-3.11**

**Description**

Technical field

The invention relates to a method and a device for in-line fat standardization of a dairy product from a separator.

Background art

Standardization means that the dairy processes the incoming whole milk, the fat content of which may vary, to make market milk with a specified constant fat content - standardized milk. If the specified constant fat content is higher than that of the incoming whole milk, the process will involve removing some of the skim milk, and the dairy will have a skim milk surplus. Normally, however, standardization proceeds to a lower fat content than that of the incoming whole milk. The process therefore leaves a surplus of fat, which can be used for example to make butter. The same type of standardization applies also for e.g. whey standardization, cream concentration, milk separation at high temperatures, where the milk is melted, or at lower temperatures, where the milk fat is solid or partly solid, or milk with ingredient addition.

The process of standardization can be arranged in three different ways: prestandardization, poststandardization, or direct standardization in line. The first step in all three methods is to separate whole milk into cream and skim milk.

Prestandardization means that the milk is standardized before being pasteurized. To adjust the fat content upward, separated cream is mixed with raw whole milk in tanks in proportions calculated to give the required fat content. To standardize to a lower fat content, the raw whole milk is diluted with separated skim milk. After analysis and adjustment, the standardized milk is pasteurized.

In poststandardization, pasteurized whole milk is mixed in tanks with either cream or skim milk, according to whether the fat content is to be adjusted upward or downward, in the same way as in the case of prestandardization. However, as poststandardization involves mixing already pasteurized products, some risk of reinfection is involved. Both methods, moreover, require the use of large, bulky tanks, and the work of analysis and adjustment is labour-intensive.

The third method, direct standardization, has therefore been an attractive alternative for industrial needs. In this method, the fat content is adjusted to the desired level by immediate remixing of a calculated proportion of the cream flow from the separator to the skim milk line.

Direct standardization begins with separation of cold or preheated whole milk into cream and skim milk of constant fat content. A regulated amount of cream is then remixed with the skim milk in an in-line system immediately after the separator to obtain standardized milk of the required fat content.

To obtain the necessary degree of precision in the process, it is necessary to be able to control all variable parameters such as fluctuations in the fat content of the incoming milk; fluctuations in throughput; fluctuations in preheating temperature.

Most of these variables are interdependent; any deviation in one step of the process therefore often results in deviations in all stages. Unless such deviations can be quickly corrected, precision of standardization will suffer and the fat content of the standardized milk will deviate from the specified value.

Performing direct standardization in a way that can handle many types of dairy products requires a rather complicated control system with online analysis of the cream flow and the skim milk flow. Such a control system includes a number of valves, connections, sensors and control equipment. One such automated direct standardization system is the Tetra Alfast sold by the Tetra Pak that can handle a variety of dairy products that has to be standardized, milk being the most common.

However, when a dairy only needs to standardize simple dairy products as milk a Tetra Alfast or similar complex machine that can control the fat content and also contents of other substances to a very exact level for many different dairy products may be unnecessary expensive. There is thus a need for a simplified automated direct standardization method for simple standardization that does not require more complex automation as e.g. Tetra Alfast. Related prior art is described in patent document US3983257A.

Summary of the invention

It is an object of the present invention to improve the current state of the art, to solve the above problems, and to provide an improvement of in-line fat standardization of a dairy product from a separator.

According to a first aspect of the invention, these and other objects are achieved in full, or at least in part, by a method according to claim 1. The method is advantageous in that it provides a simple yet reliable fat content control system using an absolute minimum of components. The step of controlling the fat content of said first processed dairy product may be further based on a known fat content of said dairy product and a known flow rate of said dairy product into said separator.

The fat content of said dairy product and said flow rate of said dairy product into said separator may be measured upstream an inlet to said separator. This way all necessary input relating to the dairy product will be available when the dairy product enters into the separator.

The valve may be controlled to a flow rate according to the following mass balance equation: FR1 = FRR * (FC1 - FCR) / (FC1 - FC2), wherein FR1 is the flow rate of said first processed dairy product; FRR is the flow rate of said dairy product; FC1 is the fat content of said first processed dairy product; FCR is the fat content of said dairy product; FC1 is the fat content of said first processed dairy product; and FC2 is the fat content of second processed dairy product. This is a preferred embodiment of the present invention and a reliable solution for providing the necessary input to the regulating valve or a control unit controlling the regulating valve. Additionally, the flow rate of said second processed dairy product may be calculated as: FR2 = FRR - FR1, wherein FR2 is the flow rate of said second processed dairy product.

According to a second aspect of the invention, these and other objects are achieved in full, or at least in part, by a device according to claim 3. The device is advantageous in that it provides a simply yet reliable fat content control system using an absolute minimum of components. If, for example, the fat content of said first processed dairy product, measured by the first fat sensor is considered too high, the flow of said first processed dairy product out of said first outlet may be regulated using the regulating valve. By limiting the flow of said first processed dairy product through the first outlet, the pressure is increased and thereby the fat content decreased.

The first fat sensor may be a mass flow meter providing both mass flow and volume flow or a density meter combined with a volume flow meter. The device may further comprise a dairy product flow meter measuring the flow rate of said dairy product entering said separator, and or a second fat sensor for detecting the fat content of said dairy product entering said separator. The detected measurements from these sensors may be used to perform calculations for the control of the valve by means of a control unit. By using a control unit, the entire control system may be automated. Thus, with the exception of a potential manual input of some standard values for the product to be processed, no manual contribution will be necessary.

When the method and device described above are used for standardizing milk, the dairy product is whole milk, the first dairy product is cream and the second dairy product is skim milk. The device for in-line fat standardization of a dairy product may be used for milk separation at high temperatures where the milk is melted or at lower temperatures where the milk fat is solid or partly solid or milk with ingredient addition.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise. The invention is defined by the appended set of claims.

Brief description of the drawings

The above objects, as well as additional objects, features and advantages of the present invention, will be more fully appreciated by reference to the following illustrative and non-limiting detailed description of preferred embodiments of the present invention, when taken in conjunction with the accompanying drawings, wherein:

Fig. 1 is a schematic drawing of a device for in-line fat standardization of a dairy product according the invention.
Fig. 2 is a block diagram showing a cascade control loop for controlling the flow rate of a first dairy product and thereby the fat content of said first dairy product.

Detailed description of preferred embodiments of the invention

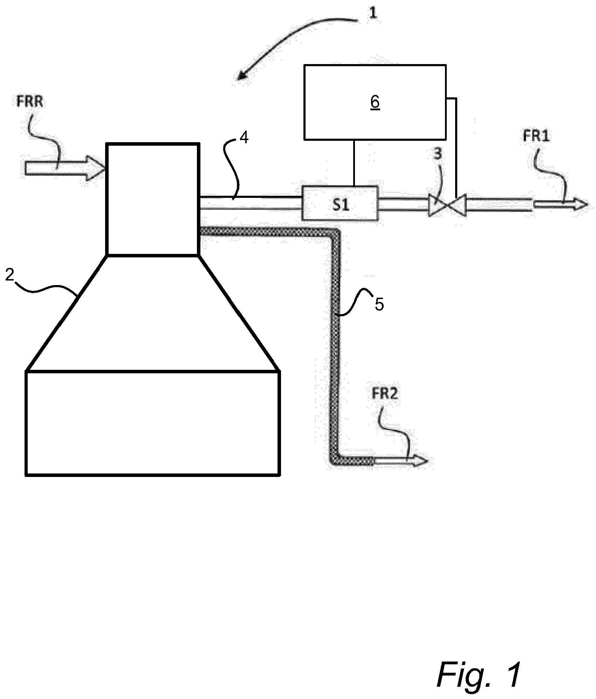

Fig. 1 shows a device 1 for in-line fat standardization of a dairy product comprising a separator 2, having an inflow of a dairy product with a flow rate FRR. The separator 2 separates the dairy product, e.g. whole milk, into a first processed dairy product flowing out through a first conduit 4 with a flow rate FR1, and a second processed dairy product flowing out through a second conduit 5 with a flow rate FR2. A valve 3 is placed on the first conduit 4, being able to throttle the flow FR1 of the first processed dairy product. A first fat sensor S1 is also arranged on the first conduit 4 able to measure fat content of the first processed dairy product flowing in the first conduit 4. The first fat sensor S1 may e.g. be a mass flow meter able to measure mass flow and volume flow or a density meter. The valve is controlled by a control unit 6 dependent on the value measured by first fat sensor S1 and a control algorithm. The control algorithm is based on a mass balance equation according to:

$$FR1 = FRR * (FC1 - FCR) / (FC1 - FC2),$$

wherein
FR1 is the flow rate of said first processed dairy product; FRR is the flow rate of said dairy product; FC1 is the fat content of said first processed dairy product; FCR is the fat content of said dairy product; FC1 is the fat content of said first processed dairy product; and FC2 is the fat content of second processed dairy product.

The fat content of the dairy product is either known for the batch that is processed from previous analysis of the dairy product and manually entered into the control unit or measured by a second fat sensor (not shown). The fat content of the second dairy product FC2 can be calculated by the fat content of the dairy product FCR and the fat content of the first processed dairy product FC1. The in-flow into the separator 2 FRR is either known from other equipment in the dairy or measured by a flow meter (not shown). The flow rate of said second processed dairy product FR2 can be calculated by the flow rate of said dairy product FRR, also referred to as the raw dairy product, and the flow rate of said first processed dairy product FR1.

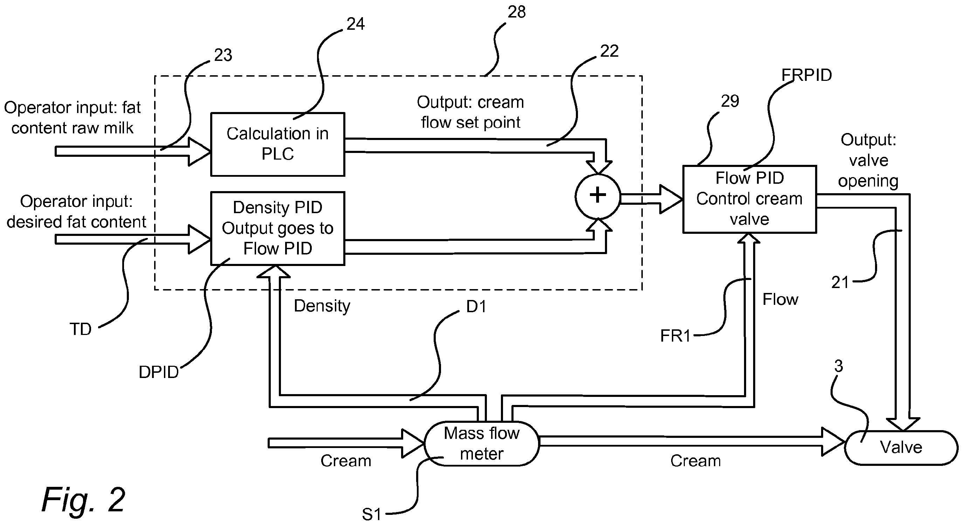

Fig. 2 shows a cascade control loop. The fat content of the first dairy product FC1 is measured by the fat sensor S1 and calculated (if needed) into a density D1 provided to a density PID regulator. A target value for the density TD of the first dairy product is also provided to the density PID regulator DPID.

The fat content of the dairy product is input to a calculation unit which will calculate an initial value for the flow rate of the first dairy product. The output signal from the density PID regulator (DPID) is provided to a flow rate PID regulator (FRPID) together with the initial value 22 for the flow rate of the first dairy product FR1. The initial value 22 is calculated 24 from the input value 23 of the flow rate of the dairy product FRR. The flow rate PID regulator (FRPID) calculates the deviation in fat content in the first dairy product compared to the target value for the density TD and provides an output signal 21 that controls the valve 3 and thereby the flow rate of the first dairy product FR1.

The control unit 6 executes the cascade control loop, and the cascade control loop comprises a primary controller 28 and a secondary controller 29. The primary controller 28 uses a value representing a density of the first processed dairy product (the cream) as control input, and the second controller 29 uses a value representing a flow rate of the first processed dairy product as control input. The primary controller 28 applies its control to the measured density against a desired density. The desired density corresponds to a desired fat content, and conversions between fat content and density is done according to known techniques and principles. Control in respect of a "fat content" may thus be understood as control in respect of a "density", and vice versa. The primary controller then provides a varying setpoint, i.e. a flow set point, to the secondary controller 29, which in turn controls the process (valve 3) directly.

Even though specific wording has been used in some parts this should not be understood as a restriction of the general concept, but as examples. For instance, the wording "raw dairy product" should be understood as any product fed into the separator and not necessarily an unprocessed milk product. The features of the method for in-line fat standardization of a dairy product may be implemented for the device for in-line fat standardization of a dairy product, and vice versa.

**Claims**

1. Method for in-line fat standardization of a dairy product from a separator (2), said separator (2) comprising an inlet for whole milk, a first outlet for cream, a second outlet for skim milk, said method comprising the steps of:

   separating whole milk, into cream flowing out through a first conduit (4) with a first flow rate (FR1), and skim milk flowing out through a second conduit (5) with a second flow rate FR2, said cream having a higher fat concentration than the skim milk,
   detecting a fat content of said cream, and
   controlling a fat content of said cream, using a valve (3) connected to said first outlet, based on said detected fat content, **characterized in that**
   said step of controlling the fat content of said cream is done using a cascade control loop, the cascade control loop comprising a primary controller (28) and a secondary controller (29),
   the primary controller (28) using a value representing a density of the cream as control input, and
   the second controller (29) using said flow rate (FR1) of the cream as control input,
   wherein said step of controlling the fat content of said cream is further based on a known fat content of said whole milk and a known flow rate of said whole milk into said separator (2), said known fat content being known from previous analysis or from measurements by a second fat sensor, said known flow rate being known from other equipment in a dairy or from measurements by a flow meter.

2. Method according to claim 1, wherein said fat content of said whole milk and said flow rate of said whole milk into said separator (2) are measured upstream an inlet to said separator (2).

3. Device for in-line fat standardization of a dairy product, comprising:

a separator (2) for separating whole milk into cream and skim milk, said separator (2) having a first outlet for said cream leaving the separator (2) at a first flow rate (FR1) and a second outlet for said skim milk leaving the separator at a second flow rate (FR2), said cream having a higher fat concentration than the skim milk

a first fat sensor (S1) for detecting a fat content of said cream at said first outlet,

a control unit (6),

a valve (3) connected to said first outlet, said valve (3) being adapted to control, by being controlled by the control unit (6), a flow of said cream out of said first outlet based on said detected fat content, **characterized by** the control unit (6) and the valve (3) being arranged to control the fat content of said cream by using a cascade control loop, the cascade control loop comprising a primary controller (28) and a secondary controller (29),

the primary controller (28) using a value representing a density of the cream as control input, and

the second controller (29) using said flow rate (FR1) of the cream as control input,

the control unit (6) being further arranged to control the fat content of said cream based on a known fat content of said whole milk and a known flow rate of said whole milk into said separator (2), said known fat content being known from previous analysis or from measurements by a second fat sensor, said known flow rate being known from other equipment in a dairy or from measurements by a flow meter.

**4.** Device according to claim 3, further comprising a whole milk flow meter measuring the flow rate of said dairy product entering said separator (2).

**5.** Device according to claim 3 or 4, further comprising a second fat sensor for detecting the fat content of said whole milk entering said separator (2).

**Patentansprüche**

**1.** Verfahren zur Inline-Fettstandardisierung eines Molkereiprodukts aus einem Separator (2), wobei der Separator (2) einen Einlass für Vollmilch, einen ersten Auslass für Sahne, einen zweiten Auslass für fettarme Milch umfasst, wobei das Verfahren die Schritte umfasst:

Trennen von Vollmilch zu Sahne, die durch eine erste Leitung (4) mit einer ersten Flussrate (FR1) abströmt, und fettarmer Milch, die durch eine zweite Leitung (5) mit einer zweiten Flussrate (FR2) abströmt, wobei die Sahne eine höhere Fettkonzentration als die fettarme Milch aufweist,

Erfassen eines Fettgehalts der Sahne und

Steuern eines Fettgehalts der Sahne unter Verwendung eines Ventils (3), das mit dem ersten Auslass verbunden ist, auf der Grundlage des erfassten Fettgehalts, **dadurch gekennzeichnet, dass**

der Schritt des Steuerns des Fettgehalts der Sahne unter Verwendung eines Kaskaden-Regelkreises durchgeführt wird, wobei der Kaskaden-Regelkreis eine primäre Steuereinheit (28) und eine sekundäre Steuereinheit (29) umfasst,

die primäre Steuereinheit (28) einen Wert, der eine Dichte der Sahne darstellt, als Steuereingang verwendet und

die zweite Steuereinheit (29) die Flussrate (FR1) der Sahne als Steuereingang verwendet,

wobei der Schritt des Steuerns des Fettgehalts der Sahne ferner auf der Grundlage eines bekannten Fettgehalts der Vollmilch und einer bekannten Flussrate der Vollmilch in den Separator (2) steht, wobei der bekannte Fettgehalt aus einer vorangehenden Analyse oder aus Messungen durch einen zweiten Fettsensor bekannt ist, wobei die bekannte Flussrate aus einer anderen Vorrichtung in einer Molkerei oder aus Messungen durch einen Durchflussmesser bekannt ist.

**2.** Verfahren gemäß Anspruch 1, wobei der Fettgehalt der Vollmilch und die Flussrate der Vollmilch in den Separator (2) stromaufwärts bezogen auf einen Einlass des Separators (2) gemessen werden.

**3.** Vorrichtung zur Inline-Fettstandardisierung eines Molkereiprodukts, umfassend:

einen Separator (2) zum Trennen von Vollmilch zu Sahne und fettarmer Milch, wobei der Separator (2) einen ersten Auslass für die Sahne, die den Separator (2) mit einer ersten Flussrate (FR1) verlässt, und einen zweiten Auslass für die fettarme Milch, die den Separator mit einer zweiten Flussrate (FR2) verlässt, aufweist, wobei die Sahne eine höhere Fettkonzentration als die fettarme Milch aufweist,

einen ersten Fettsensor (S1) zum Erfassen eines Fettgehalts der Sahne an dem ersten Auslass,

eine Steuereinheit (6),

ein Ventil (3), das mit dem ersten Auslass verbunden ist, wobei das Ventil (3) dafür ausgelegt ist, den Fluss der Sahne aus dem ersten Auslass auf der Grundlage des erfassten Fettgehalts zu steuern, wobei es von der Steuereinheit (6) gesteuert wird, **dadurch gekennzeichnet, dass**
die Steuereinheit (6) und das Ventil (3) dafür gestaltet sind, den Fettgehalt der Sahne unter Verwendung eines Kaskaden-Regelkreises zu steuern, wobei der Kaskaden-Regelkreis eine primäre Steuereinheit (28) und eine sekundäre Steuereinheit (29) umfasst,
die primäre Steuereinheit (28) einen Wert, der eine Dichte der Sahne darstellt, als Steuereingang verwendet und die zweite Steuereinheit (29) die Flussrate (FR1) der Sahne als Steuereingang verwendet,
wobei die Steuereinheit (6) ferner dafür gestaltet ist, den Fettgehalt der Sahne auf der Grundlage eines bekannten Fettgehalts der Vollmilch und einer bekannten Flussrate der Vollmilch in den Separator (2) zu steuern, wobei der bekannte Fettgehalt aus einer vorangehenden Analyse oder aus Messungen durch einen zweiten Fettsensor bekannt ist, wobei die bekannte Flussrate aus einer anderen Vorrichtung in einer Molkerei oder aus Messungen durch einen Durchflussmesser bekannt ist.

**4.** Vorrichtung gemäß Anspruch 3, ferner umfassend einen Vollmilch-Durchflussmesser, der die Flussrate des Molkereiprodukts misst, das in den Separator (2) eintritt.

**5.** Vorrichtung gemäß Anspruch 3 oder 4, ferner umfassend einen zweiten Fettsensor zum Erfassen des Fettgehalts der Vollmilch, die in den Separator (2) eintritt.

**Revendications**

**1.** Procédé de standardisation en ligne des matières grasses d'un produit laitier provenant d'un séparateur (2), ledit séparateur (2) comprenant une entrée pour du lait entier, une première sortie pour de la crème, une deuxième sortie pour du lait écrémé, ledit procédé comprenant les étapes de :

séparation de lait entier en crème s'écoulant à travers une première conduite (4) avec un premier débit (FR1), et en lait écrémé s'écoulant à travers une deuxième conduite (5) avec un deuxième débit (FR2), ladite crème ayant une plus grande concentration en matières grasses que le lait écrémé,
détection d'une teneur en matières grasses de ladite crème, et
régulation d'une teneur en matières grasses de ladite crème, au moyen d'une vanne (3) raccordée à ladite première sortie, en fonction de ladite teneur en matières grasses détectée, **caractérisé en ce que**
ladite étape de régulation de la teneur en matières grasses de ladite crème est réalisée au moyen d'une boucle de régulation en cascade, la boucle de régulation en cascade comprenant un régulateur principal (28) et un régulateur secondaire (29),
le régulateur principal (28) utilisant une valeur représentant une masse volumique de la crème comme entrée de régulation, et
le deuxième régulateur (29) utilisant ledit débit (FR1) de la crème comme entrée de régulation,
dans lequel ladite étape de régulation de la teneur en matières grasses de ladite crème est également basée sur une teneur en matières grasses connue dudit lait entier et un débit connu dudit lait entier à l'intérieur dudit séparateur (2), ladite teneur en matières grasses connue étant connue à partir d'une analyse antérieure ou à partir de mesures réalisées par un deuxième capteur de matières grasses, ledit débit connu étant connu à partir d'un autre équipement dans une laiterie ou à partir de mesures réalisées par un débitmètre.

**2.** Procédé selon la revendication 1, dans lequel ladite teneur en matières grasses dudit lait entier et ledit débit dudit lait entier à l'intérieur dudit séparateur (2) sont mesurés en amont d'une entrée dudit séparateur (2).

**3.** Dispositif pour la standardisation en ligne des matières grasses d'un produit laitier, comprenant :

un séparateur (2) pour séparer du lait entier en crème et lait écrémé, ledit séparateur (2) ayant une première sortie pour ladite crème quittant le séparateur (2) à un premier débit (FR1) et une deuxième sortie pour ledit lait écrémé quittant le séparateur à un deuxième débit (FR2), ladite crème ayant une plus grande concentration en matières grasses que le lait écrémé,
un premier capteur de matières grasses (S1) pour détecter une teneur en matières grasses de ladite crème à ladite première sortie,
une unité de régulation (6),
une vanne (3) raccordée à ladite première sortie, ladite vanne (3) étant adaptée pour réguler, en étant contrôlée par l'unité de régulation (6), un écoulement de ladite crème sortant de ladite première sortie en fonction de ladite teneur en matières grasses détectée, **caractérisé en ce que**

l'unité de régulation (6) et la vanne (3) sont agencées pour réguler la teneur en matières grasses de ladite crème en utilisant une boucle de régulation en cascade, la boucle de régulation en cascade comprenant un régulateur principal (28) et un régulateur secondaire (29),

le régulateur principal (28) utilisant une valeur représentant une masse volumique de la crème comme entrée de régulation, et

le deuxième régulateur (29) utilisant ledit débit (FR1) de la crème comme entrée de régulation,

l'unité de régulation (6) étant en outre agencée pour réguler la teneur en matières grasses de ladite crème en fonction d'une teneur en matières grasses connue dudit lait entier et d'un débit connu dudit lait entier à l'intérieur dudit séparateur (2), ladite teneur en matières grasses connue étant connue à partir d'une analyse antérieure ou à partir de mesures réalisées par un deuxième capteur de matières grasses, ledit débit connu étant connu à partir d'un autre équipement dans une laiterie ou à partir de mesures réalisées par un débitmètre.

4. Dispositif selon la revendication 3, comprenant en outre un débitmètre de lait entier mesurant le débit dudit produit laitier entrant dans ledit séparateur (2).

5. Dispositif selon la revendication 3 ou 4, comprenant en outre un deuxième capteur de matières grasses pour détecter la teneur en matières grasses dudit lait entier entrant dans ledit séparateur (2).

1

FRR

6

4

FR1

3

S1

2

5

FR2

*Fig. 1*

Fig. 2

EP 3 319 453 B1

**EP 3 319 453 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3983257 A